(19) Europäisches Patentamt
European Patent Office
Office européen des brevets

(11) Publication number: 0 205 215 B1

(12) # EUROPEAN PATENT SPECIFICATION

(45) Date of publication of patent specification: 08.08.90

(51) Int. Cl.$^5$: C 12 P 7/40, C 12 N 1/14, C 12 N 1/16, A 61 K 31/19

(21) Application number: 86200987.5

(22) Date of filing: 04.06.86

(54) **Process for the preparation of 2-arylpropionic acids.**

(30) Priority: 07.06.85 GB 8514489

(43) Date of publication of application: 17.12.86 Bulletin 86/51

(45) Publication of the grant of the patent: 08.08.90 Bulletin 90/32

(84) Designated Contracting States: AT BE CH DE FR GB IT LI LU NL SE

(56) References cited:
EP-A-0 063 731
FR-A-1 503 381
GB-A-2 004 543

CHEMICAL ABSTRACTS, vol. 83, no. 1, 7th July 1975, page 11, abstract no. 171v, Columbus, Ohio, US; W.J. WECHTER et al.: "Enzymic inversion at saturated carbon. Nature and mechanism of the inversion of R(-) p-isobutyl hydratropic acid", & BIOCHEM. BIOPHYS. RES. COMMUN. 1974, 61(3), 833-7

(73) Proprietor: GIST-BROCADES N.V.
Wateringseweg 1 P.O. Box 1
NL-2600 MA Delft (NL)
(73) Proprietor: SHELL INTERNATIONALE RESEARCH MAATSCHAPPIJ B.V.
Carel van Bylandtlaan 30
NL-2596 HR Den Haag (NL)

(72) Inventor: Phillips, Gareth Thomas
5 The Finches
Sittingbourne, Kent (GB)
Inventor: Robertson, Brian William
16 Lords Close, Bapchild
Sittingbourne, Kent ME9 8AG (GB)
Inventor: Watts, Peter Douglas
9 Woollett Road
Sittingbourne, Kent (GB)
Inventor: Matcham, George William John
12 Kestrel Close
Sittingbourne, Kent (GB)
Inventor: Bertola, Mauro Attilio
A. van Scheltemaplein 91
NL-2624 PJ Delft (NL)
Inventor: Marx, Arthur Friedrich
Fl.Nightingalelaan 12
NL-2614 GM Delft (NL)
Inventor: Koger, Hein Simon
G.Vermootenstraat 32
NL-2064 XR Spaarndam (NL)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 205 215 B1

(56) References cited:
JOURNAL OF CHROMATOGRAPHY, vol. 299, no. 2, 21st September 1984, pages 397-403, Elsevier Science Publishers B.V., Amsterdam, NL; J.M. MAITRE et al.: "High-performance liquid chromatographic separation of the enantiomers of anti-inflammatory 2-arylpropionates: suitability of the method for in vitro metabolic studies"

PATENTS ABSTRACTS OF JAPAN, vol. 7, no. 108 (C-165)1253r, 11th May 1983; & JP-A-58 29 719

AGRICULTURAL & BIOLOGICAL CHEMISTRY, vol. 45, no. 6, June 1981, pages 1389-1392, Tokyo, JP; S. IRIUCHIJIMA et al.: "Asymmetric hydrolysis of (+-)-alpha-substituted carboxylic acid esters with microorganisms"

(74) Representative: Huygens, Arthur Victor, Dr. et al
c/o Gist-Brocades N.V. Patent & Trademarks Department Martinus Nijhofflaan 2 PO Box 1
NL-2600 MA Delft (NL)

**Description**

The present invention relates to a process for the preparation of a pharmaceutically active compound in a stereospecific form of the formula

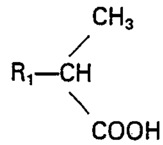

(I)

or a pharmaceutically acceptable salt or ester thereof, for example an alkali metal or an alkaline earth metal salt or a pivaloyl ester, wherein $R_1$ represents an optionally substituted aryl group such as a phenyl or naphthyl group, or represents a heterocyclic ring system, which is optionally substituted, or represents a heteroaromatic ring system containing in addition to carbon atoms, one or more atoms selected from the group consisting of nitrogen, sulphur and oxygen, this ring system being optionally substituted.

It is known that many biologically active compounds occur as a mixture of stereoisomers. Up to now these mixtures are frequently used as such in agricultural and pharmaceutical applications. Usually the desired biological activity resides in one stereoisomer so that in case of a mixture of two or more stereoisomers the potency of the mixture is at least reduced to half. Still a major reason for the use of mixtures of stereoisomers is that the cost of separation of the stereoisomers exceeds the potential advantage of a possible increase in activity. However it is apparent that modern pharmacologists are becoming increasingly aware of other implications of the administration of mixtures wherein one or more stereoisomers have to be regarded as impurities that may not have the desired therapeutic effect, but even may have other unwanted physiological effects including toxicity.

More particularly it has been found that the *in vitro* anti-inflammatory activity of naproxen as well as ibuprofen resides in the *S*-enantiomer (optionally active stereoisomer) which is up to 150 times as active as its antipode, as is known e.g. from A. Adams et al, J. Pharm. Pharmac., *28* (1976) 256 and A. J. Hutt and J. Caldwell, Clinical Pharmacokinetics 9 (1984) 371.

The selective preparation of the inactive *R*-enantioner of ibuprofen in using the microbial oxidation of 1-isobutyl-4-(1′-methyloctyl) benzene, an aromatic hydrocarbon which has an appropriate non-functional aliphatic side chain, is known from T. Sugai and K. Mori (Agric. Biol. Chem., *48* (1984) 2501). Therefore there is still a large need for a process on an industrial scale giving rise to economically attractive yields for the preparation of these *S*-enantiomers. The object of the invention is to provide such a process.

As a result of extensive research and experimentation an improved synthesis has now surprisingly been found for the preparation of particularly the *S*-enantiomers of compound (I), which comprises subjecting a compound of the formula

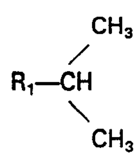

(II)

wherein $R_1$ is as defined before, to the action of a microorganism, having the ability for stereoselective oxidation of compound (II) into compound (I), having at least 70% by weight the *S*-configuration, and if desired converting compound (I) into a pharmaceutically acceptable salt or ester thereof.

More specifically the present invention relates to a process for predominantly producing a pharmaceutically active compound in stereospecific S-configuration form of formula (I) or a pharmaceutically acceptable salt or ester thereof, preferably an alkali metal salt or an alkaline earth metal salt, wherein $R_1$ is an optionally substituted aryl group such as a phenyl or naphthyl group, or represents a heterocyclic ring system, which is optionally substituted, or represents a heteroaromatic ring system containing in addition to carbon atoms, one or more atoms selected from nitrogen, sulphur and oxygen, this ring system being optionally substituted, which comprises subjecting a compound of formula (II) to the action of a micro-organism having the ability for stereo selective oxidation of compound (II) into compound (I). Preferably compound (I) is naproxen, ibuprofen, suprofen, fenoprofen, ketoprofen, benoxaprofen, carprofen, cicloprofen, pirprofen, flurbiprofen, fluprofen, tetriprofen, hexaprofen, indoprofen, mexoprofen, pranoprofen, furarofen, protizinic acid, tiaprofenic acid or brofezil.

More preferably according to the process of the present invention naproxen or ibuprofen are prepared in predominantly the *S*-configuration.

According to a preferred embodiment, the process is carried out by selecting a suitable micro-organism in such a way that compound (I) is formed thereof at least 90% by weight is in the *S*-configuration.

Suitable micro-organisms are for example fungi, yeast-like fungi and yeasts. Particularly the micro-organisms for the oxidation of compound (II) into naproxen or ibuprofen include cultures of fungi

belonging to the genus *Cordyceps* and more particularly cultures of species *Cordyceps militaris* (a strain of this species is deposited as example with CBS under the accession number of 267.85 on June 7, 1985) or a variant or mutant thereof.

In an embodiment of the invention the micro-organisms having the ability of stereoselective oxidation of compound (II) into compound (I) may be selected by using a 2-($R_1$)-alkane ($R_1$ as defined before) as the C-source in a suitable medium. Suitably a 2-(6-methoxy-2-naphthyl)alkane is used for the selection of micro-organisms having the ability for the preparation of naproxen, the alkane advantageously being pentane, heptane or nonane. A suitable medium is for example a PS III-salt medium enriched with a vitamin solution and/or 0.01% of yeast extract.

A number of micro-organisms isolated from natural sources, particularly from soil samples, were selected according to the above screening procedure, and when subjected to further tests as hereinafter described, showed their ability for stereoselective oxidation of compound (II) into compound (I).

Newly discovered micro-organisms of the invention were isolated from soil samples using 2.5 g/l 2-(6-methoxy-2-naphthyl)heptane as a C-source in PS III-salt medium enriched with a vitamine solution and/or 0.01% yeast extract.

A PS III salt medium composition contains: $KH_2PO_4$ (2.1 g/l), $(NH_4)_2HPO_4$ (1.0 g/l), $(NH_4)_2SO_4$ (0.9 g/l), KCl (0.2 g/l), $CaSO_4.2\ H_2O$ (0.005 g/l), $MgSO_4.7\ H_2O$ (0.2 g/l), $(NH_4)_2SO_4.FeSO_4.6\ H_2O$ (2.5 mg/l), $ZnSO_4.7\ H_2O$ (0.5 mg/l), $MnCl_2.4\ H_2O$ (0.3 mg/l), $CuSO_4.5\ H_2O$ (0.15 mg/l), $CoCl_2.6\ H_2O$ (0.15 mg/l), $H_3BO_3$ (0.05 mg/l), $Na_2MoO_4.2\ H_2O$ (0.055 mg/l), KI (0.1 mg/l).

The pH was adjusted to 6.8. The medium was sterilized for 20 min. at 120°C.

The composition of the vitamin solution contains: Biotin (0.002 mg/l), calciumpantothenate (0.4 mg/l), Inositol (2.0 mg/l), Nicotinic acid (0.4 mg/l), Thiamin-HCl (0.4 mg/l), Pyridoxine-HCl (0.4 mg/l), p-Aminobenzoic acid (0.2 mg/l), Riboflavin (0.2 mg/l), Folic acid, (0.01 mg/l).

The pH was adjusted to 7.0 and the medium was sterilized using a membrane filter.

The newly discovered micro-organisms were purified with agar solidified medium.

Examples of such cultures were deposited with the CBS. Micro-organisms for the oxidation of 2-(6-methoxy-2-naphthyl)propane into naproxen, obtained by the above mentioned isolation and selection method, include cultures of species Fungus 27—2, 1—2x (a strain of this species is deposited as examples with CBS under the accession number of 256.86 on May 15, 1986), species Yeast 27—1, $I_2$ (a strain of this species is deposited as example with CBS under the accession number of 257.86 on May 15, 1986), species Yeast 27—2, $II_7$ (a strain of this species is deposited as example with CBS under the accession number of 259.86 on May 15, 1986), species Yeast 22—2, $I_5$ (a strain of this species is deposited as example with CBS under the assession number of 258.86 on May 15, 1986) and variants or mutants thereof.

For the purpose of the invention the micro-organisms are advantageously used in immobilized form with a polymer gel.

According to a preferred embodiment of the process of the present invention, a micro-organism having the ability to convert compound (II) into compound (I) having at least 70% by weight the *S*-configuration, has to be cultured for about 0.5 to 10 days, whereafter the cells are suspended in a liquid nutrient medium, preferably a minimal liquid nutrient medium, and compound (II) is subjected to the action of the cells. After the abovementioned cultivation for about 0.5 to 10 days the cells may be isolated from the culturing medium before suspending the cells in the minimal liquid nutrient medium. To grow the micro-organisms used for the selective oxidation of compound (II), ordinary culture mediums containing an assimilable carbon source (for example glucose, lactate, sucrose, etc.), a nitrogen source (for example ammonium sulphate, ammonium nitrate, ammonium chloride, etc.), with an agent for an organic nutrient source (for example yeast extract, malt extract, peptone, meat extract, etc.) and an inorganic nutrient source (for example phosphate, magnesium, potassium, zinc, iron and other metals in trace amounts) may be used. As a preferred culture medium a Czapek-Dox, a BHI or a YEPD medium optionally enriched with one or more ingredients, is used.

A temperature between 0 and 45°C and a pH between 3.5 and 8 is maintained during the growth of the micro-organisms. Preferably the micro-organisms are grown at a temperature between 20 and 37°C and at a pH between 4 and 7.

The aerobic conditions required during the growth of the micro-organisms can be provided according to any of the well-established procedures, provided that the supply of oxygen is sufficient to meet the metabolic requirement of the micro-organisms. This is most conveniently achieved by supplying oxygen, preferably in the form of air. During the conversion of compound (II) into compound (I) the micro-organisms might be in a growing stage using an abovementioned ordinary culture medium.

Preferably during the conversion of compound (II) into compound (I), the micro-organisms can be held in a substantially non-growing stage using a minimal cuture medium. As minimal culture medium an ordinary culture medium may be used containing an assimilable carbon source when required (for example glucose, lactate, sucrose, etc.), a nitrogen source when required (for example ammonium sulphate, ammonium nitrate, ammonium chloride, etc.), with an agent for an organic nutrient source when required (for example yeast extract, malt extract, peptone, meat extract, etc.) and an inorganic nutrient source when required (for example phosphate, magnesium, potassium, zinc, iron and other metals in trace amounts). The micro-organisms can be kept in the non-growing state for example by exclusion of the assimilable carbon source or by exclusion of the nitrogen source. A temperature between 0 and 45°C and a

pH between 3.5 and 8 is maintained during this stage. Preferably the micro-organisms are kept at a temperature between 20 and 37°C and a pH between 4 and 7. The aerobic conditions required during this stage can be provided according to the abovementioned procedures, provided that the supply of oxygen is sufficient to meet the metabolic requirement of the micro-organisms but also to convert compound (II) into compound (I). The compound (I) produced by the micro-organisms as mentioned above, can be recovered and purified according to any of the procedures known per se for such products.

The invention will be illustrated by the following examples, however without restricting the scope of the invention.

Example I

The conversion of 2-(6-methoxy-2-naphthyl)-propane into S-2-(6-methoxy-2-naphthyl) propanoic acid using Cordyceps militaris (CBS 267.85).

Cordyceps militaris was kept on Czapek-Dox agar slants. From these it was inoculated in enriched Czapek-Dox medium of the following composition: sodium nitrate (3 g/l), dipotassium hydrogenphosphate (1 g/l), magnesium sulphate.$7H_2O$ (0.5 g/l), ferrous sulphate 7 $H_2O$ (0.01 g/l), malt extract (1 g/l), yeast extract (1 g/l) and sucrose (20 g/l), the pH adjusted to 6.5.

The fungus was grown at 25°C for 6 days in a 500 ml baffle flask containing 100 ml medium, while shaking and aerobic conditions were maintained.

To the 6 day old culture 20 mg of 2-(6-methoxy-2-naphthyl)propane dissolved in tetradecane (50 mg substrate/ml tetradecane) was added. The culture was incubated further for another 6 days at 25°C, while shaking and aerobic conditions were maintained.

For the analysis the culture was acidified to pH 2.0 with 85% $H_3PO_4$, a small amount of ammonium sulphate was added and then the mixture was extracted with dichloromethane.

The dichloromethane extracts were analysed on TLC (thin layer chromatography) plates (Merk, Precoated TLC plates Silicagel 60 F—254 with fluorescent indicator). The plates were eluted with ethylacetate-hexane (1:5 v/v). The extracts in which compounds were found with the same Rf value as Naproxen were derivatised into naphthalene methylamides and separated with HPLC.

Derivatisation procedure:

2 ml dichloromethane extract was dried under a flow of $N_2$. The dried sample was reacted with 200 µl benzene + 10 µl — thionylchloride for 10 minutes at 60°C.

The reaction mixture was dried under $N_2$.

Naphthalenemethylamine, 5%, dissolved in dried dichloromethane was then added (200 µl) and the reaction was carried on for 1 hour at room temperature. The reaction mixture was dried under $N_2$ and then extracted with 2 ml isooctane/chloroform (2:1 v/v) and 2 ml HCl, 1N.

The results of the HPLC analysis showed the presence of 8.2 mg *S*-Naproxen and 0.09 mg *R*-Naproxen per culture found. This corresponds with an enantiomeric distribution of 98.9% *S*- and 1.1% R-Naproxen.

Example II

In a parallel experiment to Example I mycelium was harvested from a 6 days old culture and reincubated in 50 ml ASM medium, while shaking and aerobic conditions were maintained. Ammonium salts medium (ASM) composition contains: ammonium chloride (0.54 g/l), potassium dihydrogen-phosphate (0.53 g/l), disodium hydrogen phosphate (0.87 g/l), potassium sulphate (0.17 g/l), magnesium sulphate. $7H_2O$ (0.07 g/l), calcium chloride.$2H_2O$ (0.073 g/l), TK3 trace element solution (1.0 ml/l) and ferrous sulphate. $7H_2O$ (0.019 g/l) added after autoclaving; the pH adjusted to 7.0. The substrate, 20 mg of 2-(6-methoxy-2-napthyl)-propane dissolved in tetradecane was added to the culture and incubated further for 6 days at 25°C. Extraction, TLC analysis, derivatisation and HPLC analysis were as described in Example I.

A total of 6.4 mg *S*-Naproxen and 0.03 mg *R*-Naproxen per culture were found. This corresponds with an enantiomeric distribution of 99.5% *S*- and 0.5% *R*-Naproxen.

Example III

Conversion of 2-(4-isobutylphenyl)-propane into S-2-(4-isobutylphenyl)propanoic acid using Cordyceps militaris (CBS 267.85)

Several 500 ml batches of *Cordyceps militaris* were grown in the supplemented Czapex-Dow medium at 25°C on a rotary shaker under aerobic conditions for five days. A Czapek Dox composition which is adapted according to "The Oxoid Manual" contains: sodium nitrate (2.0 g/l), potassium chloride (0.5 g/l), magnesium glycerophosphate (0.5 g/l), ferrous sulphate (0.01 g/l), potassium sulphate (0.35 g/l) and sucrose (30 g/l). One litre of culture broth was filtered (Whatman No. 1), resuspended in 500 ml of ASM medium and incubated with 2-(4-isobutylphenyl)propane (0.34 g, 0.4 ml) at 25°C on a shaker under aerobic conditions for five days. Incubation mixtures were filtered and the filtrate acidified to pH 2.0 with 1N HCl, and extracted with dichloromethane. The products were identified using GLC which indicated the presence of the required acid. The methylene chloride was extracted with sodium bicarbonate which was acidified with 1N HCl and re-extracted into methylene chloride. The solvent was evaporated to give the crude product of 2-(4-isobutylphenyl)-propanoic acid. The impure product was purified in 30 mg batches using a silicagel column prepared in hexane and eluted with 5% diethyl ether in hexane. S-2-(4-isobutylphenyl)-propanoic acid was obtained which analysed as a single component by GLC and TLC.

In total 43 mg of *S*-2-(4-isobutylphenyl)propanoic acid was obtained which analysed as a single component by GLC an TLC and whose PMR spectrum was consistent with the racemic compound; PMR ($CDCl_3$) δ: 0.9 (d, 6H, CH—($CH_3)_2$); 1.5 (d, 3H, CH—$CH_3$); 1.85 (m, 1H, *CH*—($CH_3)_2$); 2.45 (d, 2H, —$CH_2$—); 3.7 (q, 1H, —*CH*—$CH_3$); 7.1—7.25 (, 4H, aromatic). Traces of impurity were removed by preparative HPLC using a Spherisorb S5NH column (25 cm × 4.9 mm i.d.) eluting with 20% isopropanol in hexane/dichloromethane (60/40, v/v) at 2 ml/min.

Repeated injections were used to collect 4 mg of *S*-2-(4-isobutylphenyl)propanoic acid eluting at 3.2 min.

The enantiomeric purity of *S*-2-(4-isobutylphenyl)propanoic acid was determined by analysing the composition of the diastereoisomeric amides formed with *S*-(−)-α-methylbenzylamine. The results of the HPCL analysis showed the presence of 90% diastereoisomer corresponding to *S*-2-(4-isobutylphenyl)-propanoic acid at retention time 2.89 min and 10% diastereoisomer corresponding to *R*-2-(4-isobutylphenyl)propanoic acid at retention time 2.48 min.

## Example IV

Preparative scale transformation using Cordyceps militaris. (CBS 267.85)

The general procedure for the production of 2-(6-methoxy-2-naphthylpropanoic acid involved growing several batches of the organism on Czapek-Dox medium supplemented with 0.1% malt extract and 0.1% yeast extract for 5 days at 25°C. The mycelium from 2 × 500 ml cultures was filtered (Whatman no. 1 paper), resuspended in 500 ml of ammonium salts medium and treated with 200 mg of 2-(6-methoxy-2-naphthyl)-propane in 2 ml of dimethylformamide. In some instances the mycelium from one 500 ml culture was filtered and resuspended in 500 ml medium with 100 mg of 2-(6-methoxy-2-naphthyl)propane in 1 ml of dimethylformamide. Incubations were kept at 25°C for 6 days on a rotary shaker-incubator.

The filtrate from the incubations was acidified to pH 2.0 with 2N HCl and extracted with methylene chloride which was extracted with 1% $NaHCO_3$ to remove the acids. The bicarbonate extract was acidified to pH 2.0 with 2N HCl and the precipitate acids recovered by filtration. The crude acids were purified in batches whereby ca 30—50 mg of impure 2-(6-methoxy-2-naphthyl)propanoic acid were purified on a column (1 cm × 7 cm) of silicagel (mesh 60—120) prepared in hexane. The pure 2-(6-methoxy-2-naphthyl)propanoic acid was eluted using diethylether in hexane (3/7, v/v) and checked for purity using the aforementioned Thin Layer Chromatography system. Pure samples of the acid were recrystallised from acetone/hexane.

Conversion of 2-(6-methoxy-2-naphthyl)propane into S-2-(6-methoxy-2-naphthyl)propanoic acid using Cordyceps militaris (CBS 267.85)

The conversion of 800 mg of 2-(6-methoxy-2-naphthyl)propane gave 50 mg of bicarbonate extract which after chromatography gave 40 mg of pure *S*-2-(6-methoxy-2-naphthyl)propanoic acid analysing as one component by glc and tlc, with $[\alpha]_D^{25}$ = +58.1°, (C=1.24, $CHCl_3$). Recrystallisation gave material with m.p. 152—153°C (lit. m.p. 152—154°C); $[\alpha]_D^{25}$ = +62.2° (C=1.08, $CHCl_3$), (lit. $[\alpha]_D^{25}$ = +66°); pmr ($CHCl_3$): δ 1.6 (d, 3H, CH—$CH_3$) 3.92 (S, 3H, O$CH_3$) 3.88 (q, 1H, *CH*) and 7—8 (m, 6H, aromatic) identical to commercial *S*-Naproxen;

Fast Atom Bombardment mass spectra gave signals at m/z 231 $[M+H]^+$, 185 $[M—HCO_2H + H]^+$, 323 $[M + 6 + H]^+$, 115 $[G + Na]^+$ and 229 $(M—H]^-$, 321 $[M + G - H]^-$ (G=glycerol).

Measurement of optical purity of R and S-2-(6-methoxy-2-naphthyl)propanoic acids by conversion into the diastereoisomeric amides with S-(−)-α-methylbenzylamine and separation by HPLC.

The 2-(6-methoxy-2-naphthyl)propanoic acid isolated from *Cordyceps militaris* was converted into the amide using *S*-(−)-α-methylbenzylamine under conditions that have been described. The product was analyzed using HPLC, as explained hereafter, which gave the following results: chromatographic composition of diastereoisomers formed was 3.4% *R*-2-(6-methoxy-2-naphthyl)propionamide (5.73 min) and 96.6% *S*-2-(6-methoxy-2-naphthyl)propionamide (6.78 min); commercial Naproxen gave 5% *R*-2-(6-methoxy-2-naphthyl)propionamide (4.08 min) and 95% *S*-2-(6-methoxy-2-naphthyl)propionamide (4.83 min).

High performance liquid chromatographic system for separating the diastereoisomeric amides formed using R,S, R or S-2-(6-methoxy-2-naphthyl)propanoic acid with R- or S-α-methylbenzylamine.

A Gilson Isocratic HPCL system was used with ultra violet detection at 250 nm. A Spherisorb S5NH column (25 cm × 4.9 mm i.d.) was employed under ambient conditions. The mobile phase, 25% (dichloromethane-isopropanol, 9:1) in hexane was used at a flow rate of 2 ml/min. Typical retention times were 4.0 min for the *S,R* and *R,S* diastereoisomers and 4.8 min for *S,S* and *R,R* diastereoiomers.

## Example V

The strains Fungus 27—2, 1—2x (CBS 256.86), Yeast 27—1, $I_2$ (CBS 257.86), Yeast 22—2, $I_5$ (CBS 258.86) and Yeast 27—2, $II_7$ (CBS 259.86) were tested for their potential to oxidise the 2-(6-methoxy-2-naphthyl)-propane. The strains were grown in 100 ml culture medium for 48 hours at 30°C. Then 20 mg 2-(6-methoxy-2-naphthyl)propane dissolved in 0.4 ml tetradecane was added to the cultures and incubated further for 3 to

4 days. At each time point twice 200 ml culture was extracted for each micro-organism with dichloromethane. The naproxen formed was derivatized into naphthalenemethylamides and analyzed with HPLC (see Example 1).

TABLE 1

| Organism | Medium* used | Time point (days) | naproxen** formed mg/l | % S | %R |
|---|---|---|---|---|---|
| Fungus 27—2, 1—2x | Czapek-Dox | 3 | 3.7 | 98 | 2 |
| (CBS 256.86) | | 4 | 7.8 | 95 | 5 |
| Yeast 27—1, $I_2$ | YEPD | 3 | 4.2 | 81 | 19 |
| (CBS 257.86) | | 4 | 6.9 | 84 | 16 |
| Yeast 22—2, $I_5$ | BHI | 3 | 2.6 | 83 | 17 |
| (CBS 258.86) | | 4 | 1.9 | 83 | 17 |
| Yeast 27—2, $II_7$ | YEPD | 3 | 2.9 | 72 | 28 |
| (CBS 259.86) | | 4 | 3.1 | 72 | 28 |

* Czapek-Dox medium as in Example I

Composition of Brain-Heart Infusion (BHI): 37 g/1 BHI (Oxoid ®), the pH was adjusted to 7.0 and the medium was sterilized for 20 min. at 120°C.
Composition of YEPD medium: 10 g/l yeast extract, 20 g/l Bactopepton, 20 g/l glucose, the pH was adjusted to 7.0 and the medium was sterilized for 30 min. at 110°C.
** The value indicated is the amount naproxen found after derivatisation. Each quantity represents the mean of two values obtained.

**Claims**

1. A process for the preparation of a pharmaceutically active compound in a stereospecific form of the formula

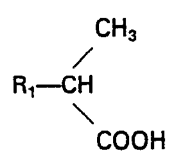

(I)

or a pharmaceutically acceptable salt or ester thereof, for example an alkali metal or an alkaline earth metal salt or a pivaloyl ester, wherein $R_1$ represents an optionally substituted aryl group such as a phenyl or naphthyl group, or represents a heterocyclic ring system, which is optionally substituted, or represents a heteroaromatic ring system containing in addition to carbon atoms, one or more atoms selected from the group consisting of nitrogen, sulphur and oxygen, this ring system being optionally substituted, which comprises subjecting a compound of the formula

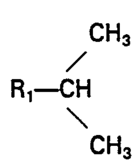

(II)

to the action of a microorganism having the ability for stereoselective oxidation of compound (II) into compound (I), having at least 70% by weight the *S*-configuration, and if desired converting compound (I) into a pharmaceutically acceptable salt or ester thereof.

2. A process according to claim 1 wherein compound (I) is naproxen, ibuprofen, cicloprofen, suprofen, carprofen, ketoprofen, benoxaprofen, fenoprofen, pirprofen, flurbiprofen, fluprofen, tetripropfen, hexaprofen, indoprofen, mexoprofen, pranoprofen, furaprofen, protizinic acid, tiaprofenic acid or brofezil.

3. A process according to claim 2 wherein compound (I) is naproxen or ibuprofen.

4. A process according to claims 1—3 wherein said microorganism is able to convert a compound (II) into a compound (I), having at least 90% by weight the *S*-configuration.

5. A process according to claims 1—4, wherein said microorganism is a fungus belonging to the genus *Cordyceps.*

6. A process according to claim 5, wherein said microorganism is *Cordyceps militaris*, preferably *Cordyceps militaris* (CBS 267.85) or a variant or mutant thereof.

7. A process according to claims 1—4, wherein said microorganism is isolated and selected from natural sources by using a 2-($R_1$)-alkane as a C-source in a suitable medium.

8. A process according to claim 7, wherein $R_1$ represents a 6-methoxy-2-naphthyl group and the alkane being preferably pentane, heptane or nonane.

9. A process according to claims 1—4 or 8, wherein said microorganism is a fungus, preferably *Fungus 27—2, 1—2x* (CBS 256.86) or a variant or mutant thereof.

10. A process according to claims 1—4 or 8, wherein said microoganism is a yeast or a yeast-like fungus.

11. A process according to claim 10, wherein said microoganism is *Yeast 27—1,* $I_2$ (CBS 257.86) or a variant or mutant thereof.

12. A process according to claim 10, wherein said microoganism is *Yeast 27—2,* $II_7$ (CBS 259.86) or a variant or mutant thereof.

13. A process according to claim 10, wherein said microorganism is *Yeast 22—2,* $I_5$ (CBS 258.86) or a variant or mutant thereof.

14. A process according to any of the preceding claims wherein said microorganism is immobilized into polymer gel.

15. A process for the isolation and selection from natural sources of microorganisms having the ability for the stereoselective oxidation of a compound of the formula

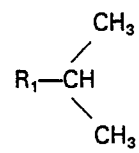

(II)

wherein $R_1$ represents an optionally substituted aryl group such as a phenyl or naphthyl group, or represents a heterocyclic ring system, which is optionally substituted, or represents a heteroaromatic ring system containing in addition to carbon atoms, one or more atoms selected from nitrogen, sulphur and oxygen, this ring system being optionally substituted, into a compound of the formula

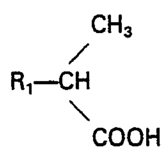

(I)

having at least 70% by weight of the *S*-configuration, by using a 2-($R_1$)alkane as C-source in a suitable medium.

16. A process according to claim 15 wherein $R_1$ represents a 6-methoxy-2-naphthyl group and the alkane is preferably pentane, heptane or nonane.

17. Fungus 27—2, 1—2x (CBS 256.86).

18. Yeast 27—1, $I_2$ (CBS 257.86).

19. Yeast 27—2, $II_7$ (CBS 259.86).

20. Yeast 22—2, $I_5$ (CBS 258.86).

**Patentansprüche**

1. Verfahren zur Herstellung einer pharmazeutisch aktiven Verbindung der Formel:

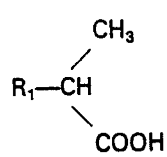

(I)

in einer stereospezifischen Form oder eines pharmazeutisch unbedenklichen Salzes oder Esters davon, z.B. eines Alkalimetallsalzes oder eines Erdalkalimetallsalzes oder eines Pivaloylesters, worin $R_1$ eine gegebenenfalls substituierte Arylgruppe, wie eine Phenyl- oder Naphthylgruppe, darstellt oder ein heterocyclisches Ringsystem darstellt, das gegebenenfalls substituiert ist, oder ein heteroaromatisches

Ringsystem darstellt, das zusätzlich zu Kohlenstoffatomen ein oder mehrere Atome enthält, die aus der Gruppe gewählt sind, die aus Stickstoff, Schwefel und Sauerstoff besteht, wobei dieses Ringsystem gegebenenfalls substituiert ist, dadurch gekennzeichnet, dass man eine Verbindung der Formel:

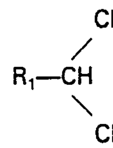 (II)

der Einwirkung eines Mikroorganismus mit der Fähigkeit zur stereoselektiven Oxidation von Verbindung (II) zu Verbindung (I), von der mindestens 70 Gew.-% die S-Konfiguration haben, unterwirft und gewunschtenfalls Verbindung (I) in eine pharmazeutisch unbedenkliches Salz oder einen pharmazeutisch unbedenklichen Ester davon überführt.

2. Verfahren nach Anspruch 1, dadurch gekennzeichnet, dass Verbindung (I) Naproxen, Ibuprofen, Cicloprofen, Suprofen, Carprofen, Ketoprofen, Benoxaprofen, Fenoprofen, Pirprofen, Flurbiprofen, Fluprofen, Tetriprofen, Hexaprofen, Indoprofen, Mexoprofen, Pranoprofen, Furaprofen, Protizinic acid, Tiaprofenic acid oder Brofezil ist.

3. Verfahren nach Anspruch 2, dadurch gekennzeichnet, dass Verbindung (I) Naproxen oder Ibuprofen ist.

4. Verfahren nach den Ansprüchen 1 bis 3, dadurch gekennzeichnet, dass der Mikroorganismus eine Verbindung (II) in eine Verbindung (I), von der mindestens 90 Gew.-% die S-Konfiguration haben, überzuführen vermag.

5. Verfahren nach den Ansprüchen 1 bis 4, dadurch gekennzeichnet, dass der Mikroorganismus ein Pilz ist, der zu der Gattung Cordyceps gehört.

6. Verfahren nach Anspruch 5, dadurch gekennzeichnet, dass der Mikroorganismus Cordyceps militaris, vorzugsweise Cordyceps militaris (CBS 267.85), oder eine Variante oder Mutante davon ist.

7. Verfahren nach den Ansprüchen 1 bis 4, dadurch gekennzeichnet, dass der Mikroorganismus aus natürlichen Quellen unter Verwendung eines 2-($R_1$)-Alkans als C-Quelle in einem geeigneten Medium isoliert und selektioniert wird.

8. Verfahren nach Anspruch 7, dadurch gekennzeichnet, dass $R_1$ eine 6-Methoxy-2-naphthylgruppe bedeutet und das Alkan vorzugsweise Pentan, Heptan oder Nonan ist.

9. Verfahren nach den Ansprüchen 1 bis 4 oder 8, dadurch gekennzeichnet, dass der Mikroorganismus eine Pilz, vorzugsweise Fungus 27—2, 1—2x (CBS 256.86), oder eine Variante oder Mutante davon ist.

10. Verfahren nach den Ansprüchen 1 bis 4 oder 8, dadurch gekennzeichnet, dass der Mikroorganismus eine Hefe oder ein hefeähnlicher Pilz ist.

11. Verfahren nach Anspruch 10, dadurch gekennzeichnet, dass der Mikroorganismus Yeast 27—1, $I_2$ (CBS 257.86) oder eine Variante oder Mutante davon ist.

12. Verfahren nach Anspruch 10, dadurch gekennzeichnet, dass der Mikroorganismus Yeast 27—2, $II_7$ (CBS 259.86) oder eine Variante oder Mutante davon ist.

13. Verfahren nach Anspruch 10, dadurch gekennzeichnet, dass der Mikroorganismus Yeast 22—2, $I_5$ (CBS 258.86) oder eine Variante oder Mutante davon ist.

14. Verfahren nach einem der vorangehenden Ansprüche, dadurch gekennzeichnet, dass der Mikroorganismus in Polymergel immobilisiert wird.

15. Verfahren zur Isolierung und Selektion von Mikroorganismen mit der Fähigkeit zur stereoselektiven Oxidation einer Verbindung der Formel:

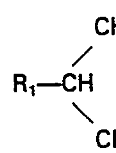 (II)

worin $R_1$ eine gegebenenfalls substituierte Arylgruppe, wie eine Phenyl- oder Naphthylgruppe, darstellt oder ein heterocyclisches Ringsystem darstellt, das gegebenenfalls substituiert ist, oder ein heteroaromatisches Ringsystem darstellt, das zusätzlich zu Kohlenstoffatomen ein oder mehrere Atome enthält, die aus Stickstoff, Schwefel und Sauerstoff gewählt sind, wobei dieses Ringsystem gegebenenfalls substituiert ist, zu einer Verbindung der Formel:

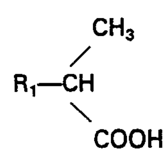 (I)

von der mindestens 70 Gew.-% die S-Konfiguration haben, aus natürlichen Quellen durch Verwendung eines 2-($R_1$)-Alkans als C-Quelle in einem geeigneten Medium.

16. Verfahren nach Anspruch 15, dadurch gekennzeichnet, dass $R_1$ eine 6-Methoxy-2-naphthylgruppe darstellt und das Alkan vorzugsweise Pentan, Heptan oder Nonan ist.

17. Fungus 27—2, 1—2x (CBS 256.86).

18. Yeast 27—1, $I_2$ (CBS 257.98).

19. Yeast 27—2, $II_7$ (CBS 259.86).

20. Yeast 22—2, $I_5$ (CBS 258.86).

## Revendications

1. Procédé de préparation d'un composé pharmaceutiquement actif sous une forme stéréospécifique de la formule

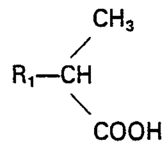

(I)

ou d'un sel ou ester pharmaceutiquement acceptable de celui-ci, par exemple un sel de métal alcalin ou un sel de métal alcalino-terreux ou un ester pivaloylique, où $R_1$ représente un radical aryle éventuellement substitué tel qu'on radical phényle ou naphthyle, ou représente un système hétérocyclique qui est éventuellement substitué, ou représente un système hétéroaromatique contenant en plus des atomes de carbone un ou plusieurs atomes choisis dans la classe formée par l'azote, le soufre et l'oxygène, ce système étant éventuellement substitué, qui comprend l'exposition d'un composé de formule

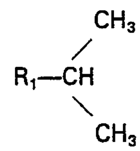

(II)

à l'action d'un micro-organisme ayant l'aptitude à l'oxydation stéréosélective du composé (II) en le composé (I), ayant pour au moins 70% en poids la configuration *S*, et si la chose est souhaitée, la conversion du composé (I) en un sel ou ester pharmaceutiquement acceptable de celui-ci.

2. Procédé suivant la revendication dans lequel le composé (I) est le naproxène, l'ibuprofène, le cicloprofène, le suprofène, le carprofène, le kétoprofène, le bénoxaprofène, le fénoprofène, le pirprofène, le flurbiprofène, le fluprofène, le tétriprofène, l'hexaprofène, l'indoprofène, le mexoprofène, le pranoprofène, le furaprofène, l'acide protizinique, l'acide tiaprofénique ou le brofézil.

3. Procédé suivant la revendication 2, dans lequel le composé (I) est le naproxène ou l'ibuprofène.

4. Procédé suivant les revendications 1—3, dans lequel le micro-organisme est apte à convertir un composé (II) en un composé (I) ayant pour au moins 90% en poids la configuration *S*.

5. Procédé suivant les revendications 1—4, dans lequel le micro-organisme est un champignon appartenant au genre *Cordyceps.*

6. Procédé suivant la revendication 5, dans lequel le micro-organisme est *Cordyceps militaris*, de préférence *Cordyceps militaris* (CBS 267.85) ou un variant ou mutant de celui-ci.

7. Procédé suivant les revendications 1—4, dans lequel le micro-organisme est isolé et sélectionné à partir de sources naturelles en utilisant un 2-($R_1$)-alcane comme source de carbone dans un milieu approprié.

8. Procédé suivant la revendication 7, dans lequel $R_1$ représente un radical 6-méthoxy-2-naphtyl et l'alcane est de préférence le pentane, l'heptane ou le nonane.

9. Procédé suivant les revendications 1—4 ou 8, dans lequel le micro-organisme est un champignon de préférence *Champignon 27—2, 1—2x* (CBS 256.86) ou un variant ou mutant de celui-ci.

10. Procédé suivant les revendications 1—4 ou 8, dans lequel le micro-organisme est une levure ou un champignon semblable à un levure.

11. Procédé suivant la revendication 10, dans lequel le micro-organisme est *Levure 27—1, $I_2$* (CBS 257.86) ou un variant ou mutant de celle-ci.

12. Procédé suivant la revendication 10, dans lequel le micro-organisme est *Levure 27—2, $II_7$* (CBS 259.86) ou un variant ou mutant de celle-ci.

13. Procédé suivant la revendication 10, dans lequel le micro-organisme est *Levure 22—2 $I_5$* (CBS 258.86) ou un variant ou mutant de celle-ci.

14. Procédé suivant l'une quelconque des revendications précédentes, dans lequel le microorganisme est immobilisé dans un gel de polymère.

15. Procédé d'isolement et de sélection à partir de sources naturelles de micro-organismes ayant l'aptitude à l'oxydation stéréosélective d'un composé de formule

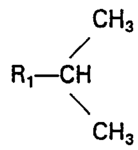

(II)

où $R_1$ représente un radical aryle éventuellement substitué tel qu'on radical phényle ou naphtyle, ou représente un système hétérocyclique qui est éventuellement substitué, ou représente un système hétéroaromatique contenant en plus des atomes de carbone un ou plusieurs atomes choisis parmi l'azote, le soufre et l'oxygène, ce système étant éventuellement substitué, en un composé de formule

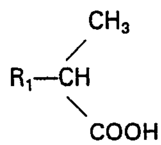

(I)

ayant pour au moins 70% en poids la configuration *S*, en utilisant un 2-($R_1$)alcane comme source de carbone dans un milieu approprié.

16. Procédé suivant la revendication 15, dans lequel $R_1$ représente un radical 6-méthoxy-2-naphtyle et l'alcane est de préférence le pentane, l'heptane ou le nonane.

17. Le Champignon 27—2, 1—2x (CBS 256.86).

18. La Levure 27—1, $I_2$ (CBS 257.86).

19. La Levure 27—2, $II_7$ (CBS 259.86).

20. La Levure 22—2 $I_5$ (CBS 258.86).